# EUROPEAN PATENT APPLICATION

(11) **EP 2 196 185 A1**
(43) Date of publication of application: **16.06.2010**
(21) Application number: 08792764.6
(22) Date of filing: 27.08.2008
(51) Int. Cl.: A61K 8/25, A61K 8/11, A61K 8/894, A61Q 13/00, A61Q 19/00, B01J 13/04

(54) **COMPOSITION CONTAINING VESICLE-SILICA COMPLEX, AND PROCESS FOR PRODUCTION THEREOF**

(30) Priority: 31.08.2007 JP 2007226827
(71) Applicant: Shiseido Company, Ltd., Chuo-ku Tokyo 104-8010 (JP)
(72) Inventor: WATANABE, Kei, Yokohama-shi Kanagawa 224-8558 (JP); NAGARE, Yuko, Yokohama-shi Kanagawa 224-8558 (JP)
(74) Representative: Lippert, Stachow & Partner
(86) International application number: PCT/JP2008/065254
(87) International publication number: WO 2009/028528

(57) **Abstract**

To provide a vesicle-containing composition wherein the stability is improved and the rate of releasing drug or perfume is sufficiently restricted during administration in vivo or application to the skin surface.
Silica polymerization on the outer surfaces of vesicles is facilitated by adding a specifically structured water-soluble silane derivative to an aqueous composition containing the vesicles composed of a surfactant and mixing them together, and vesicle-silica composites having silica-coated outer surfaces can be obtained, resulting in the improved stability of the vesicle-containing composition as well as the improved effect of restricting the rate of releasing drug or perfume during administration in vivo or application to the skin surface.

## Description

### RELATED APPLICATIONS

This application claims the priority of Japanese Patent Application No. 2007-226827 filed on August 31, 2007, which is incorporated herein by reference.

### FIELD OF THE INVENTION

The present invention relates to a vesicle-silica composite-containing composition and the production method thereof, and in particular, relates to improved stability of a composition which contains vesicles composed of a surfactant.

### BACKGROUND OF THE INVENTION

Among amphiphilic compounds with both hydrophilicity and hydrophobicity, there are compounds, such as phospholipids, that form a bilayer membrane (lamellar phase) spherical structure in an aqueous phase. Such bilayer membrane structures are called liposomes or vesicles. An aqueous component can be incorporated inside the vesicle, and an oil component can be incorporated in the vesicle membrane. Thus, when incorporating a drug or a perfume into a vesicle, the vesicle restricts its release rate. For example, when a vesicle is administered in a living subject, the metabolism of the drug is restricted, and the drug efficacy can be retained for a longer period. When a perfume-incorporating vesicle is applied to the skin surface, the scent from the perfume can be retained for a longer period. Because of these advantages, a vesicle is used as a microcapsule in fields such as pharmaceuticals, cosmetics, and food. Additionally, because the formation of liposomes or vesicles can provide a composition with a good appearance (transparency) and a good feeling in use, they are expected to be used as base of cosmetics.

In recent years, silicone surfactants have been reported as amphiphilic compounds that can form such vesicles (for example, see Patent Literatures 1 to 3). The characteristics of the vesicles formed by a silicone surfactant are, for example, that the vesicles can be more easily prepared in comparison with the case in which other vesicle forming surfactants are used. However, because the vesicles prepared with a silicone surfactant have high membrane fluidity, they cannot achieve sufficient temporal stability or temperature stability, resulting in insufficient effect of restricting the rate of releasing a drug or a perfume during administration in vivo or application to the skin surface. In addition, such vesicles have a problem that, depending on the kinds of other coexisting substances, they collapse easily. Especially when considering using such vesicles as a base for cosmetics, there has been demand for the improved stability enough for enduring an actual use.

Patent Literature 1: Japanese Unexamined Patent Publication H07-323222.
Patent Literature 2: Japanese Unexamined Patent Publication H08-239475.
Patent Literature 3: Japanese Unexamined Patent Publication H09-175930.

### DISCLOSURE OF THE INVENTION

### PROBLEM TO BE SOLVED BY THE INVENTION

The present invention was made in view of the above-described problems of the conventional art, and the objects are to provide a vesicle-containing composition
wherein the stability is improved and the rate of releasing drug or perfume is sufficiently restricted during administration in vivo or application to the skin surface.

### MEANS TO SOLVE THE PROBLEM

The present inventors have diligently studied to solve the above-described problems of the conventional art. As a result, the present inventors have found that silica polymerization on the outer surfaces of vesicles is facilitated by adding a specifically structured water-soluble silane derivative to an aqueous composition containing the vesicles composed of a surfactant and mixing them together. Vesicle-silica composites having silica-coated outer surfaces can be obtained, resulting in the improved stability of the vesicle-containing composition as well as the improved effect of restricting the rate of releasing a drug or a perfume during administration in vivo or application to the skin surface, thus leading to completion of the present invention.

The present invention provides a vesicle-silica composite-containing composition comprising: (A) a surfactant which can form vesicles in water and (B) water, and containing a vesicle-silica composite wherein a part or a whole of outer surface of the vesicles composed of the (A) surfactant is coated with silica.

In addition, in the vesicle-silica composite-containing composition, it is preferred that the (A) surfactant is a polyoxyalkylene-modified silicone.
In addition, in the vesicle-silica composite-containing composition, it is preferred that the amount of the (A) surfactant is 0.3 to 5.0 % by mass with respect to the total amount of the composition. In addition, the vesicle-silica composite-containing composition preferably contains a vesicle-silica composite particle having two or more vesicle particles in an aggregated state bonded by silica.

The present invention provides a production method of the vesicle-silica composite-containing composition comprising the steps of: mixing ingredients containing (A) a surfactant which can form vesicles in water and (B) water to form vesicles composed of the (A) surfactant, and adding (C) a water-soluble silane derivative represented by following formula (1) to the vesicle-containing aqueous composition obtained in the previous step to form silica on a part or a whole of outer surface of the vesicles composed of the (A) surfactant.

Si-(OR¹)₄ (1)

In the formula (1), at least one of R¹s is a polyhydric alcohol residue and the rest can be alkyl groups.

In the production method of the vesicle-silica composite-containing composition, it is preferred that the (A) surfactant is a polyoxyalkylene-modified silicone.
In the production method of the vesicle-silica composite-containing composition, it is preferred that the amount of the (A) surfactant is 0.3 to 5.0 % by mass with respect to the total amount of the composition.
In the production method of the vesicle-silica composite-containing composition, it is preferred that the amount of the (C) water-soluble silane derivative is 1.0 to 10.0 % by mass with respect to the total amount of the composition.
In the production method of the vesicle-silica composite-containing composition, it is preferred that a vesicle-silica composite particle having two or more vesicle particles in an aggregated state bonded by silica is formed.

The present invention provides a cosmetic comprising the vesicle-silica composite-containing composition.
The present invention provides a fragrance cosmetic comprising the vesicle-silica composite-containing composition.

### EFFECT OF THE INVENTION

According to the present invention, silica polymerization on the outer surfaces of vesicles is facilitated by adding a specifically structured water-soluble silane derivative to an aqueous composition containing the vesicles composed of a surfactant and mixing them together, and vesicle-silica composites having silica-coated outer surfaces can be obtained, resulting in the improved stability of the vesicle-containing composition as well as the improved effect of restricting the rate of releasing drug or perfume during administration in vivo or application to the skin surface.

### BRIEF DESCRIPTION OF THE DRAWINGS

Fig. 1 shows pictures of the vesicle-containing compositions prepared by using various concentrations of the polyoxyethylene-modified silicone and a glycerin-substituted silane derivative.
Fig. 2 is a view showing size variations of particles in the vesicle-containing composition with respect to concentrations of the water-soluble silane derivative.
Fig. 3 shows the result of measuring the size change of the particles before and after addition of the surfactant, in the vesicle-silica composite according to one example of the present invention.
Fig. 4 shows the result of measuring the size change of the particles before and after addition of the surfactant, in a untreated vesicle.
Fig. 5 is a TEM micrograph of the vesicle-silica derivative (singly-dispersed vesicle) prepared with 5 % by mass of the surfactant and 0.1 % by mass of the water-soluble silane derivative.
Fig. 6 is a TEM micrograph of the vesicle-silica composite (vesicle aggregate) prepared with 5 % by mass of the surfactant and 3.0 % by mass of the water-soluble silane derivative.

### BEST MODE FOR CARRYING OUT THE INVENTION

The vesicle-silica composite-containing composition according to the present invention is characterized by comprising: (A) a surfactant which can form vesicles in water and (B) water, and containing a vesicle-silica composite wherein a part or a whole of outer surface of the vesicles composed of the (A) surfactant is coated with silica.

### (A) surfactant

The (A) surfactant used in the present invention is not particularly restricted as far as it can form vesicles in water. As such (A) surfactant which can form vesicles in water, for example, a polyoxyalkylene-modified silicone can be preferably used. A polyoxyalkylene-modified silicone is a surfactant having a polysiloxane structure as the hydrophobic group and a polyoxyalkylene structure as the hydrophilic group. A specific example of such a polyoxyalkylene-modified silicone includes the one represented by the following formula (2).

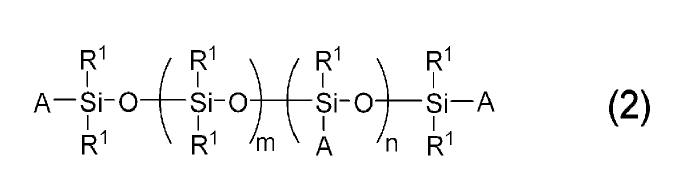

In the formula (2), R¹ represents a hydrogen atom or an alkyl group having 1 to 6 carbon atoms, as for A, at least one of As is a polyoxyalkylene group represented by the formula: -(CH₂)ₐ-(C₂H₄O)_{b}-(C₃H₆O)_{c}-R², the other As are hydrogen atom(s) or alkyl group(s) having 1 to 6 carbon atoms, the number m represents an integer of 1 to 200, and n represents an integer of 0 to 50; and in the formula: -(CH₂)ₐ-(C₂H₄O)_{b}-(C₃H₆O)_{c}-R², R² represents a hydrogen atom or an alkyl group having 1 to 6 carbon atoms, a represents an integer of 1 to 6, b represents an integer of 0 to 50, c represents an integer of 0 to 50, and b + c is at least 5 or higher.

In the above formula (2), R¹ is a side chain on the backbone polysiloxane structure, and it is a hydrogen atom or an alkyl group having 1 to 6 carbon atoms. These may be either same with or different from each other. For example, when all R¹s are methyl groups, the structure is a dimethylpolysiloxane structure, and when R¹s are a methyl group and a phenyl group, the structure is a methylphenylpolysiloxane structure. The group A locates where polyoxyalkylene groups can be introduced on the backbone of the polysiloxane structure, and at least one of the groups A is a polyoxyalkylene group represented by the formula: -(CH₂)ₐ-(C₂H₄O)_{b}-(C₃H₆O)_{c}-R² (in the formula, R² represents a hydrogen atom or an alkyl group having 1 to 6 carbon atoms, a represents an integer of 1 to 6, b represents an integer of 0 to 50, c represents an integer of 0 to 50, and b + c is at least 5 or higher).

In the above formula (2), when a portion of the groups A is the above polyoxyalkylene group, the other groups A can be hydrogen atoms or an alkyl groups having 1 to 6 carbon atoms. For example, when two terminal groups A are polyoxyalkylene groups, the polyoxyalkylene-modified silicone is an ABA-type. On the other hand, when only the non-terminal groups A are polyoxyalkylene groups, the polyoxyalkylene-modified silicone is a pendant-type. The polyoxyalkylene group can be any one of the following: polyoxyethylene group, polyoxypropylene group, and polyoxyethylene/polyoxypropylene group. The number of moles of the non-substituted polysiloxane structure m is 1 to 200. The number of moles of the polyoxyalkylene-substituted polysiloxane structure n is 0 to 50. When n is 0, it is necessary that either one or both of the two terminal groups A are polyoxyalkylene groups.

Specific examples of the polyoxyalkylene-modified silicone used in the present invention include polyoxyethylene (12 mol) modified dimethylpolysiloxane (pendant-type polyoxyalkylene-modified silicone wherein the side chain methyl group of a linear dimethylpolysiloxane is replaced with a polyoxyethylene (12 mol) group), polyoxyethylene (8 mol) modified dimethylpolysiloxane, and polyoxyethylene (20 mol) modified dimethylpolysiloxane. Other examples include an ABA-type polyoxyethylene-methylsiloxane-polyoxyethylene block copolymer. In these polyoxyethylene-modified silicones, it is preferable that the molecular weight of ethylene oxide in the total molecular weight is 20 to 60%. The polyoxyalkylene-modified silicone used in the present invention can be produced by a publicly known method, or commercial products can be used. Examples of the commercial polyoxyalkylene-modified silicones include SH3772M, SH3773M, SH3775M (all manufactured by Dow Corning Silicone Co., Ltd.), and IM-22 (manufactured by Wacker Chemical Corp.). These polyoxyalkylene-modified silicones can be used alone or more than one can be arbitrarily selected and used in combination.

The vesicle-silica composite-containing composition of the present invention contains the (A) surfactant as vesicles. The formation of vesicles can be easily carried out by a publicly known method. For example, the vesicles composed of the (A) surfactant can be formed in an aqueous formulation by adding the (A) surfactant to the aqueous formulation containing the (B) water and mixing them. To allow the (A) surfactant to be dissolved easily, it is preferred that an appropriate amount of aqueous solvent such as ethanol, propylene glycol, dipropylene glycol, and 1,3-butylene glycol is blended in the aqueous formulation. The vesicle-silica composite particle size is not limited in particular, however, it is normally about 20 to 500 nm and preferably 50 to 200 nm. The measurement of the average particle size can be conducted with, for example, dynamic light scattering method, laser diffractometry, and so on.

The (A) surfactants can be used either singly or in combination of two or more.
The amount of the (A) surfactant is not limited in particular as long as it is enough for the formation of vesicles. However, the amount is preferably 0.1 to 10.0 % by mass of the total amount of the composition, and more preferably 0.3 to 5.0 % by mass. If the amount of the (A) surfactant is small, the effect of vesicle formation may not be achieved. On the other hand, if the amount is too large, the vesicle stability may be poor.

### (B) Water

The amount of the (B) water is not limited in particular, however, the amount (i.e., the total amount of water added before and after the vesicle formation) is preferably 70.0 to 90.0 % by mass of the total amount of the composition, and more preferably 80 to 95 % by mass. If the amount of the (B) water is small, vesicles may not be formed. On the other hand, if the amount of the (B) water is too large, the relative amount of the (A) surfactant or any component incorporated in the vesicles decrease. As a result, the effect of the vesicle formation or the effect of blending the component incorporated in the vesicles may not be achieved.

### Production method

The vesicle-silica composite-containing composition according to the present invention comprises the above (A) surfactant and the (B) water, and it can be obtained as a composition containing the vesicle-silica composite with silica-coated outer surface by adding the (C) water-soluble silane derivative having a specific structure to the aqueous composition containing the vesicles composed of the (A) surfactant and mixing them together to facilitate silica polymerization on the outer surface of the vesicles.

Hereinafter, the production method is described in more detail by explaining one example method of producing the vesicle-silica composite-containing composition of the present invention.
1) First, ingredients including the (A) surfactant which can form vesicles in water and the (B) water are mixed to form the vesicles composed of the (A) surfactant.
   To allow the (A) surfactant to be dissolved easily, it is preferred that an appropriate amount of aqueous solvent such as ethanol, propylene glycol, dipropylene glycol, and 1,3-butylene glycol is blended in addition to the above components (A) and (B). In the formation of the vesicles, it is also possible to mix the above components (A) and (B) together with any aqueous component or oil component as a vesicle-incorporating component.

The vesicles can also be formed by dissolving the (A) surfactant which can form vesicles in water into a solvent such as chloroform, evaporating the chloroform in a container such as an eggplant-type flask to obtain a thin film of the (A) surfactant, adding the (B) water, and then conducting a ultrasonication.

2) Consequently, the (C) water-soluble silane derivative represented by the following formula (1) is added to the vesicle-containing aqueous composition obtained in the previous step to form silica on a part or a whole of the outer surface of the vesicles composed of the (A) surfactant.

Si-(OR¹)₄ (1)

In the formula (1), at least one of R¹s is a polyhydric alcohol residue and the rest can be alkyl groups.

### (C) Water-soluble silane derivative

The (C) water-soluble silane derivative used in the present invention is represented by the above formula (1). In the water-soluble silane derivative represented by the above formula (1), at least one of R¹s is a polyhydric alcohol residue and the rest can be alkyl groups. The polyhydric alcohol residue is represented in a form wherein one hydroxyl group is removed from a polyhydric alcohol. The (C) water-soluble silane derivative can usually be prepared by a substitution reaction of tetraalkoxysilane with a polyhydric alcohol. The polyhydric alcohol residue R¹ varies depending upon the kinds of polyhydric alcohols. For example, R¹ is -CH₂-CH₂-OH when ethylene glycol is used as the polyhydric alcohol. Furthermore, at least one of R¹s may be a substituted polyhydric alcohol residue and the rest can be unsubstituted alkyl groups.

Examples of the polyhydric alcohol residues R¹ in the above formula (1) include an ethylene glycol residue, a diethylene glycol residue, a triethylene glycol residue, a tetraethylene glycol residue, a polyethylene glycol residue, a propylene glycol residue, a dipropylene glycol residue, a polypropylene glycol residue, a butylene glycol residue, a hexylene glycol residue, a glycerin residue, a diglycerin residue, a polyglycerin residue, a neopentyl glycol residue, a trimethylolpropane residue, a pentaerythritol residue, and a maltitol residue. Among these, it is preferable that R¹ is one selected from the group consisting of an ethylene glycol residue, a propylene glycol residue, a butylene glycol residue, and a glycerin residue.

More specific examples of the (C) water-soluble silane derivative used in the present invention include Si-(O-CH₂-CH₂-OH)₄, Si-(O-CH₂-CH₂-CH₂-OH)₄, Si-(O-CH₂-CH₂-CHOH-CH₃)₄, and Si-(O-CH₂-CHOH-CH₂-OH)₄.

The (C) water-soluble silane derivative used in the present invention can be prepared, for example, by the reaction of a tetraalkoxysilane and a polyhydric alcohol in the presence of a solid catalyst.

The tetraalkoxysilane may be any so far as four alkoxy groups are bonded to a silicon atom, and it is not limited in particular. Examples of the tetraalkoxysilanes for the production of water-soluble silicate monomers include tetramethoxysilane, tetraethoxysilane, tetraisopropoxysilane, tetrapropoxysilane, and tetrabutoxysilane. Among these, the use of tetraethoxysilane is the most preferable from the viewpoint of availability and the safety of reaction by-products.

As an alternative compound for the tetraalkoxysilane, a mono-, di-, or trihalogenated alkoxysilane such as monochlorotriethoxysilane, dichlorodimethoxysilane, or monobromotriethoxysilane; or a tetrahalogenated silane such as tetrachlorosilane could be used. However, these compounds generate strong acids such as hydrogen chloride and hydrogen bromide during the reaction with a polyhydric alcohol. As a result, the corrosion of the reaction apparatus may take place, and the post-reaction separation and removal are difficult; thus they are not practicable.

The polyhydric alcohol may be any compound as long as two or more hydroxyl groups are present in the molecule, and it is not limited in particular. Examples of the polyhydric alcohols used for the production of water-soluble silicate monomers include ethylene glycol, diethylene glycol, triethylene glycol, tetraethylene glycol, polyethylene glycol, propylene glycol, dipropylene glycol, polypropylene glycol, butylene glycol, hexylene glycol, glycerin, diglycerin, polyglycerin, neopentyl glycol, trimethylolpropane, pentaerythritol, and maltitol. Among these, it is preferable to use one selected from the group of ethylene glycol, propylene glycol, butylene glycol, and glycerin.

The solid catalyst may be any as long as it is a solid catalyst insoluble in raw materials, reaction solvent, and reaction products and it is a solid having active acid points and/or base points for the substituent exchange reaction on the silicon atom. Examples of the solid catalysts used in the present invention include ion exchange resins and various inorganic solid acid/base catalysts.

Examples of the ion exchange resins used as the solid catalyst include acidic cation exchange resins and basic anion exchange resins. Examples of matrix resins for these ion exchange resins include styrene-type, acryl-type, and methacryl-type resins. Examples of functional groups with catalyst activity include sulfonic acid, acrylic acid, methacrylic acid, quaternary ammonium, tertiary amine, and primary and secondary polyamines. The matrix structure of ion exchange resin can be selected, depending upon the objectives, from the group consisting of a gel type, a porous type, and a biporous type.

Examples of the acidic cation exchange resins include Amberlite IRC76, FPC3500, IRC748, IRB120B Na, IR124 Na, 200CT Na (manufactured by Rohm and Haas Co.), Diaion SK1B, PK208 (manufactured by Mitsubishi Chemical Corporation), Dowex Monosphere 650C, Marathon C, HCR-S, and Marathon MSC (manufactured by Dow Chemical Company). Examples of the basic anion exchange resins include Amberlite IRA400J Cl, IRA402BL Cl, IRA410J Cl, IRA411 Cl, IRA458RF Cl, IRA900J Cl, IRA910CT Cl, IRA67, IRA96SB (manufactured by Rohm and Haas Co.), Diaion SA10A, SAF11AL, SAF12A, PAF308L (manufactured by Mitsubishi Chemical Corporation), Dowex Monosphere 550A, Marathon A, Marathon A2, and Marathon MSA (manufactured by Dow Chemical Company).

Inorganic solid acid/base catalysts used as the solid catalyst are not limited in particular. Examples of the inorganic solid acid catalysts include single metal oxides such as Al₂O₃, SiO₂, ZrO₂, TiO₂, ZnO, MgO, and Cr₂O₃; composite metal oxides such as SiO₂-Al₂O₃, SiO₂-TiO₂, SiO₂-ZrO₂, TiO₂-ZrO₂, ZnO-Al₂O₃, Cr₂O₃-Al₂O₃, SiO₂-MgO, and ZnO-SiO₂; metal sulfates such as NiSO₄ and FeSO₄; metal phosphates such as FePO₄; immobilized sulfuric acid such as H₂SO₄/SiO₂; immobilized phosphoric acid such as H₂PO₄/SiO₂; immobilized boric acid such as H₃BO₃/SiO₂; natural minerals or layered compounds such as activated clay, zeolite, kaolin, and montmorillonite; synthetic zeolite such as AlPO₄-zeolite; and heteropolyacids such as H₃PW₁₂O₄₀ · 5H₂O and H₃PW₁₂O₄₀. Examples of the solid inorganic base catalysts include single metal oxides such as Na₂O, K₂O, Rb₂O, Cs₂O, MgO, CaO, SrO, BaO, La₂O₃, ZrO₃, and ThO₃; metal salts such as Na₂CO₃, K₂CO₃, KHCO₃, KNaCO₃, CaCO₃, SrCO₃, BaCO₃, (NH₄)₂CO₃, Na₂WO₄ · 2H₂O, and KCN; metal oxide-supported alkali metals such as Na-Al₂O₃ and K-SiO₂; zeolite-supported alkali metals such as Na-mordenite; and composite metal oxides such as SiO₂-MgO, SiO₂-CaO, SiO₂-SrO, SiO₂-ZnO, SiO₂-Al₂O₃, SiO₂-ThO₂, SiO₂-TiO₂, SiO₂-ZrO₂, SiO₂-MoO₃, SiO₂-WO, Al₂O₃-MgO, Al₂O₃-ThO₂, Al₂O₃-TiO₂, Al₂O₃-ZrO₂, ZrO₂-ZnO, ZrO₂-TiO₂, TiO₂-MgO, and ZrO₂-SnO₂.

A solid catalyst can be easily separated, by the treatment such as filtration or decantation, from the products after the completion of the reaction.

In the production of the (C) water-soluble silane derivative, it is not necessary to use a solvent during the reaction; however, various solvents may be used as necessary. The solvent used in the reaction is not limited in particular, and the examples include aromatic hydrocarbons such as benzene, toluene, and xylene; ester, ether, and ketone solvents such as ethyl acetate, methyl acetate, acetone, methyl ethyl ketone, Cellosolve, diethyl ether, and dioxane; polar solvents such as acetonitrile, dimethylformamide, and dimethyl sulfoxide; and halogenated solvents such as chloroform and dichloromethane. In order to suppress the hydrolysis-condensation reaction of a tetraalkoxysilane, which is used as a raw material, it is desirable to dehydrate the solvent in advance. Among these solvents, it is preferable to use acetonitrile, toluene, etc. that can promote the reaction by removing alcohols, such as ethanol, formed as a reaction by-product to the outside of the system by forming an azeotropic mixture.

The (C) water-soluble silane derivative may be used either alone or in combination of two or more. The amount of the (C) water-soluble silane derivative is not limited in particular, and it is preferably 0.5 to 10.0 % by mass of the total amount of the composition, more preferably 1.0 to 10.0 % by mass. If the amount of the water-soluble silane derivative is less than 0.5 % by mass, the stabilizing effect of vesicle may not be achieved. If it exceeds 10.0 % by mass, the vesicle-silica composite particles become too large and they separate and precipitate, resulting in a poor feeling in use.

Silica gel and polyhydric alcohol result from hydrolysis and dehydration-condensation reaction of the (C) water-soluble silane derivative in water. During this reaction, if there is any substance which can be an appropriate scaffold in water, the formation of silica gel is facilitated by using the substance as scaffold. Consequently, silica polymerization on the outer surface of the vesicles is facilitated by adding the (C) water-soluble silane derivative to the aqueous composition containing the vesicles composed of the (A) surfactant and mixing them together, and a composition containing the vesicle-silica composites wherein the outer surface is coated with silica can be obtained.

Depending on the amount of the above (C) water-soluble silane derivative, the type of the obtained composites varies as follows: (1) a singly-dispersed vesicle-silica composite, that is a vesicle-silica composite wherein one vesicle particle is coated with silica, and (2) a vesicle aggregate-silica composite, that is a vesicle-silica composite particle wherein two or more vesicle particles in an aggregated state are bonded with silica. Both types can be useful for the vesicle-silica composite-containing composition of the present invention, however, in particular, the latter type (2): vesicle-silica composite having vesicle aggregate structure can be preferably used. If the amount of the (C) water-soluble silane derivative exceeds 1.0 % by mass, the (2) vesicle aggregate-silica composite are likely to be obtained in particular.

The vesicle-silica composite-containing composition of the present invention, which is obtained as mentioned above, can suitably used, for example, as cosmetics. When the composition is used as cosmetics, other components normally used in pharmaceuticals and cosmetics can be arbitrarily blended in addition to the above (A) to (C) essential components so far as the amount is in the range that does not affect the vesicle or silica formation and stability thereof. These other formulation components can be blended before vesicle formation, after vesicle formation, or after vesicle-silica composite formation. For example, a microcapsule composition wherein any chemical component is present only in the inner phase (or in the vesicle bilayer membrane) can be prepared by forming vesicles by blending the aqueous (or oil) chemical component into a formulation and then replacing the outer phase.

The use application of the cosmetics according to the present invention is not limited in particular, and they are suitably used as general cosmetics such as a lotion and a hair liquid. In particular, it is possible to preferably use the cosmetics as fragrance cosmetics expected for the effect of restricting perfume release.

### EXAMPLES

Hereinafter, the present invention will be described in further detail with reference to the examples. However, the present invention is not limited by these examples. Hereinafter, the amount is expressed in mass % unless otherwise noted.

First, the method of producing the water-soluble silane derivative used in the present invention will be explained.

### Synthesis Example 1: Glycerin-substituted silane derivative

60.1 g (0.28 mol) of tetraethoxysilane and 106.33 g (1.16 mol) of glycerin were mixed, and 1.1 g of strong acid ion exchange resin (Dowex 50W-X8, manufactured by the Dow Chemical Company) was added as a solid catalyst. This mixture without a solvent was mixed with stirring at 85 °C. After about 3 hours, the mixture became a one-layer transparent solution. After the reaction was continued for an additional 5 hours 30 minutes, the obtained solution was allowed to stand overnight. Under reduced pressure, the solid catalyst was separated by filtration and washed with a small amount of ethanol. From this solution, ethanol was distilled away, and 112 g of transparent viscous liquid was obtained. The product formed a uniform transparent gel, with a slight exotherm, by mixing with the same amount of water at room temperature (yield: 97%).

The present inventors prepared a glycerin-substituted silane derivative according to the above synthesis example, tried preparing vesicle-silica composite by blending the glycerin-substituted silane derivative into a solution containing the vesicles composed of a surfactant, and investigated the obtained product.

### Vesicle-silica composite

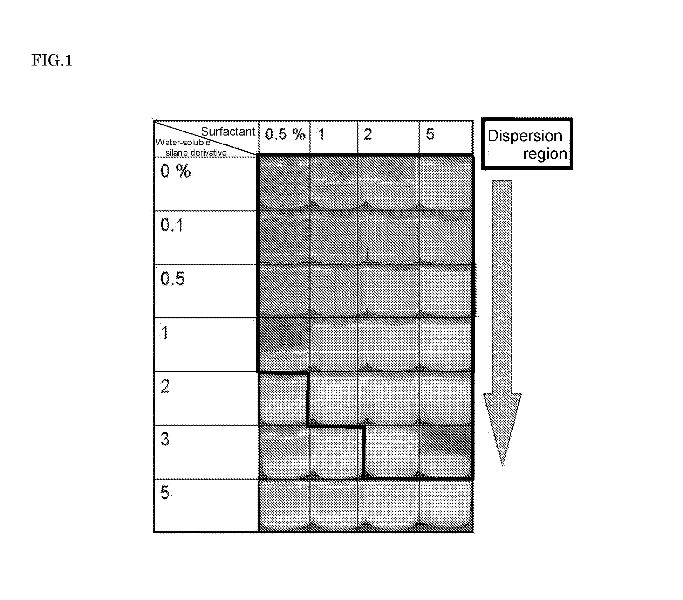

10 mass parts of polyoxyethylene (12 mol) dimethylpolysiloxane (SH3773M, manufactured by Dow Corning Toray Co., Ltd.) as a surfactant was dissolved in 10 mass parts of ethanol. The mixture was further dissolved in 80 mass parts of water to obtain an aqueous solution containing the vesicles composed of polyoxyethylene modified silicone. Then, the solution was diluted with 10 % by mass of ethanol solution, and several vesicle-containing solutions wherein surfactant concentrations were respectively 0.5, 1.0, 2.0, and 5.0 % by mass were obtained. To each of the obtained vesicle-containing solutions with different surfactant concentrations, 0.1, 0.5, 1.0, 2.0, 3.0, and 5.0 % by mass of the glycerin-substituted silane derivatives were added respectively, and they were allowed to stand at room temperature for one week.
The micrographs of the various vesicle-containing compositions obtained as mentioned above are shown in Fig. 1.

As shown in Fig. 1, when each concentration of the surfactant and the water-soluble silane derivative was low, the composition was in a dispersed state having almost transparent appearance. However, the system became cloudy as these concentrations increased and exceeded 1.0 % by mass, and precipitation was brought about as these concentrations became even higher.

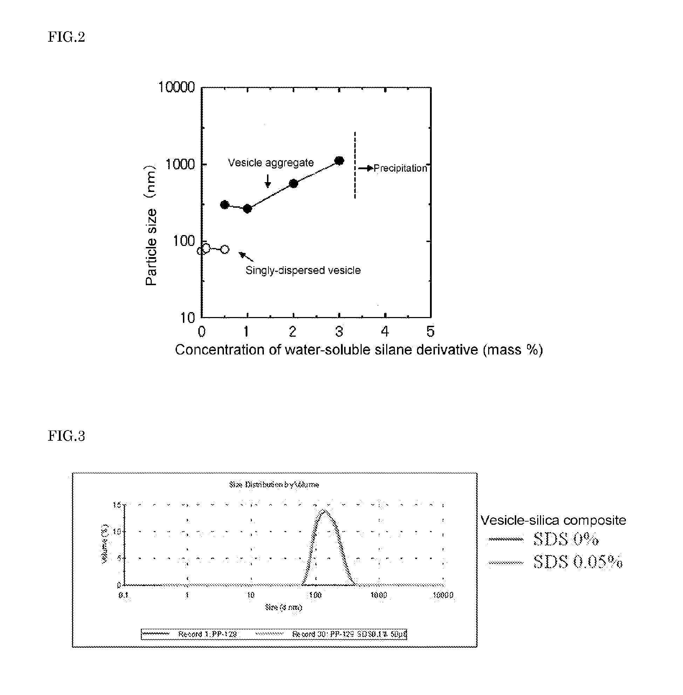

Additionally, in the above example, various vesicle-containing compositions were prepared with using 5 % by mass of the surfactant and appropriately changed concentrations of the water-soluble silane derivatives, and each particle size in the obtained vesicle-containing compositions was measured with dynamic light scattering method (Zetasizer Nano-ZS, manufactured by Malvern Instruments).
The particle size variation with respect to the concentration of the water-soluble silane derivative is shown in Fig. 2.

As shown in Fig. 2, when the concentration of the water-soluble silane derivative was less than 0.5 % by mass, only the particles having about 100 nm size could be observed, thus it was found that the vesicles were dispersed singly. On the other hand, when the concentration of the water-soluble silane derivative was 0.5 % by mass, two sizes of particles, some particles about 100 nm in size and some particles about a few hundred nm in size were observed, thus it was thought that the larger particles were aggregates of vesicles. Additionally, when the concentration of the water-soluble silane derivative increased to 1.0 % by mass or more, only the aggregates having a few hundred nm size were observed. The size of the aggregates increased depending on the concentration of the water-soluble silane derivative, and precipitation was brought about when the concentration exceeded 3 % by mass.

According to these results, it can be thought as follows: in the area that the concentration of the water-soluble silane derivative is low, silica is preferentially formed at the periphery of a singly-dispersed vesicle particle, which is dispersed in water as a vesicle-silica composite; on the other hand, several vesicle particles are bonded by silica to form an aggregate when the concentration of the water-soluble silane derivative becomes high and exceeds 1.0 % by mass, and when the aggregate becomes too large, it can no longer disperse, resulting in precipitation.

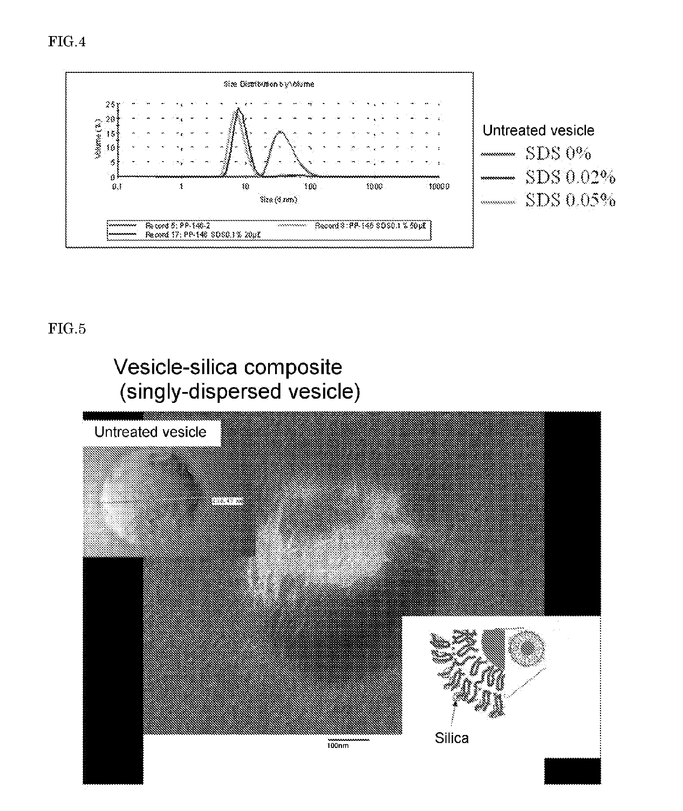

Subsequently, a vesicle-silica composite-containing composition was prepared using 0.5 % by mass of the surfactant and 1.0 % by mass of the water-soluble silane derivative in the same manner with the above test, and 0.02 and 0.05 % by mass of sodium dodecyl sulfate were respectively added to the composition. The particle sizes before and after adding the sodium dodecyl sulfate were measured by dynamic light scattering method in each mixture. As comparative examples, the same test was conducted with use of a vesicle-containing solution without the water-soluble silane derivative.
The result of measuring the particle size of the vesicle-silica composite is shown in Fig. 3 , that of the untreated vesicle is shown in Fig. 4.

As shown in Figs. 3 and 4, it can be found that, in the untreated vesicle, the vesicle structure collapsed by adding 0.02 % by mass of the sodium dodecyl sulfate as a surfactant, and the particle size decreased from approximately 40 nm to a few nm. On the contrary, in the vesicle-silica composite, the particle size was about a few hundred nm which was larger than that of the untreated vesicle, and the particle size did not change at all even after adding 0.05 % by mass of the sodium dodecyl sulfate.

According to this result, it is thought that silica is formed on the outer surface of the vesicle composed of the surfactant by adding the water-soluble silane derivative, and silica serves as if being film coating, resulting in significantly stabilized vesicle structure.

Additionally, transmission electron microscope (TEM) micrographs of the vesicle-silica composite prepared in the above test were taken in a freeze replica method, using H-7000: manufactured by Hitachi, Ltd.

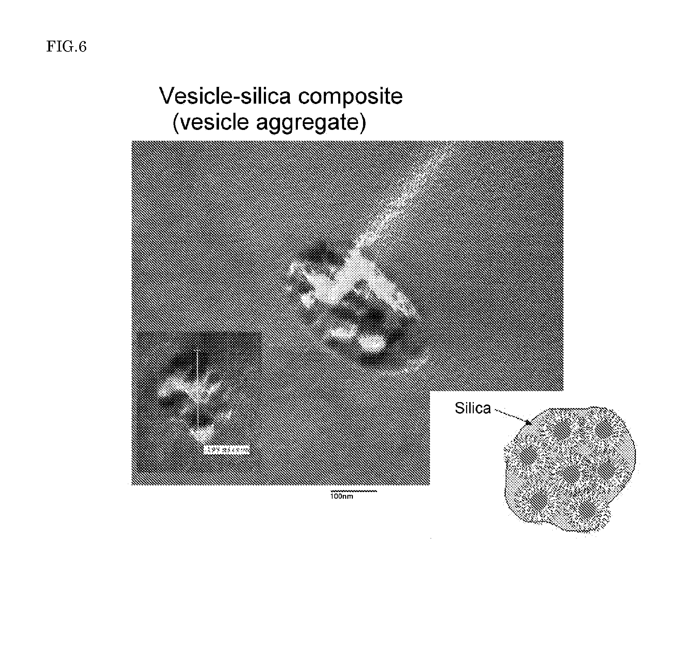

Fig. 5 shows a TEM micrograph of the vesicle-silica derivative (singly-dispersed vesicle) prepared with use of 5 % by mass of the surfactant and 0.1 % by mass of the water-soluble silane derivative. Fig. 6 shows a TEM micrograph of the vesicle-silica composite (vesicle aggregate) prepared with use of 5 % by mass of the surfactant and 3.0 % by mass of the water-soluble silane derivative.

As shown in Fig. 5, it can be understood that the singly-dispersed vesicle-silica composite has a larger particle size compared with the untreated vesicle, and the outer surface of the vesicle particle is coated with silica.
As shown in Fig. 6, in the vesicle-silica composite prepared with use of increased concentration (1.0 % by mass or more) of the water-soluble silane derivative, it can be observed that a plurality of vesicle particles are present within one vesicle-silica composite particle, that is a plurality of vesicle particles in an aggregated state are bonded by silica to form one vesicle-silica composite particle.

### Cosmetics

Subsequently, the present inventors tried preparing cosmetics (lotions) using the above-explained vesicle-silica composite and evaluated them in terms of appearance, feeling in use, vesicle stability, and long-lasting effect of scent from perfume. As comparative examples, cosmetics were prepared by blending the untreated vesicle and fine-particle silica gel, and they were evaluated similarly. Table 1 shows compositions of the cosmetics used in the test and the evaluation results. The evaluation criteria are as follows.

### (1) Appearance

The appearance of the cosmetic (lotion) in each example and comparative example was visually evaluated.

### (2) Feeling in use

The actual use test by 10 professional panelists was conducted for the feeling in use (permeation to skin, spreadability during application, and sticky feeling) when the cosmetic (lotion) in each example and comparative example was used. The evaluation criteria are as follows.

### <Evaluation criteria>

⊚: 8 or more panelists recognized that the feeling in use was good.
○: 6 or more and less than 8 panelists recognized that the feeling in use was good.
△: 3 or more and less than 6 panelists recognized that the feeling in use was good.
X: less than 3 panelists recognized that the feeling in use was good.

### (3) Vesicle stability

A surfactant (sodium dodecyl sulfate) was added to the cosmetic (lotion) in each example and comparative example, and the vesicle stability was evaluated by measuring the vesicle particle sizes before and after adding the surfactant. The evaluation criteria are as follows.

### <Evaluation Criteria>

⊚: There is no change in vesicle particle sizes before and after adding the surfactant.
○: There is very little change in vesicle particle sizes before and after adding the surfactant.
X: There is a change in vesicle particle sizes before and after adding the surfactant.

### (4) Long-lasting effect of scent from perfume

The actual use test by 10 professional panelists was conducted for long-lasting effect of scent from perfume when the cosmetic (lotion) in each example and comparative example was used. The evaluation criteria are as follows.
⊚: 8 or more panelists recognized that the scent was long-lasting.
○: 5 or more and less than 8 panelists recognized that the scent was long-lasting.
X: less than 5 panelists recognized that the scent was long-lasting.

**Table 1**

| | Example 1 | Example 2 | Comparative Example 1 | Comparative Example 2 | Comparative Example 3 | Comparative Example 4 |
|---|---|---|---|---|---|---|
| PEG(12)dimethylpoly siloxane (HLB=8) | 0.3 | 0.3 | 0.3 | 0.3 | 0.3 | 0.3 |
| Water | Balance | Balance | Balance | Balance | Balance | Balance |
| Dipropylene glycol | 5.0 | 5.0 | 5.0 | 5.0 | 5.0 | 5.0 |
| Glycerin-substituted silane derivative | 0.5 | 5.0 | - | - | - | - |
| Fine-particle silica | - | - | 0.05 | 0.3 | - | - |
| Glycerin | - | - | 0.45 | 4.7 | 0.45 | 4.7 |
| Limonene | 0.05 | 0.05 | 0.05 | 0.05 | 0.05 | 0.05 |
| (1) Appearance | Transparent and dispersed | Cloud and dispersed | Separated and precipitated | Separated and precipitated | Transparent and dispersed | Transparent and dispersed |
| (2) Feeling in use | ⊚ | ⊚ | X | X | ○ | ○ |
| (3) Vesicle stability | ○ | ⊚ | X | X | X | X |
| (4) Long-lasting effect of scent from perfume | ○ | ⊚ | X | X | X | X |

(Production method) PEG(12)dimethylpolysiloxane, water, dipropylene glycol, and perfume were mixed to form vesicles, the glycerin-substituted silane derivative was added thereto, and the mixture was allowed to stand. Then, the rest of the components and water were added to the mixture to prepare a lotion.

It was found that, as shown in Table 1, each lotion of Examples 1 and 2 was in a dispersed state having a transparent or cloud appearance and excellent in the feeling in use, the vesicle stability, and the long-lasting effect of the scent from perfume. On the contrary, in each lotion of Comparative Examples 1 and 2, in which the glycerin-substituted water-soluble silane derivative in Examples 1 and 2 was replaced by fine-particle silica and glycerin, the fine-particle silica separated to precipitate, resulting in a poor feeling in use, an insufficient vesicle stability, and an insufficient long-lasting effect of the scent from perfume. Each of Comparative Examples 3 and 4, in which a glycerin was substituted for the whole glycerin-substituted water-soluble silane derivative in Examples 1 and 2, had a transparent appearance and a relatively good feeling in use, however, it had a poor vesicle stability and a poor long-lasting effect of the scent from perfume.

Hereinafter, the present invention will be described in further detail with reference to other examples of the cosmetic of the present invention. However, the present invention is not limited by these examples.

| Formulation Example 1 Fragrance | (mass %) |
|---|---|
| PEG-12 dimethicone (HLB=8) | 10 |
| Perfume | 0.8 |
| Decamethylcyclopentasiloxane | 3.2 |
| Ethanol | 5 |
| Glycerin-substituted silane derivative | 3 |
| 1,3-Butylene glycol | 2 |
| EDTA · 3Na · 2H₂O | 0.05 |
| Citric acid | 0.02 |
| Sodium citrate | 0.08 |
| Water | Balance |

The fragrance obtained in the above formulation example 1 had a transparent appearance and was excellent in the feeling in use, the vesicle stability, and the long-lasting effect of the scent from perfume.

| Formulation Example 2 Lotion | (mass %) |
|---|---|
| PEG-12 dimethicone (HLB=8) | 0.3 |
| Dipropylene glycol | 2 |
| Glycerin-substituted silane derivative | 3 |
| EDTA · 3Na · 2H₂O | 0.05 |
| Citric acid | 0.02 |
| Sodium citrate | 0.08 |
| Vitamin E acetate | 0.01 |
| PEG-400 | 0.05 |
| Vitamin C | 1 |
| Water | Balance |

The lotion obtained in the above formulation example 2 had a transparent appearance and was excellent in the feeling in use and the vesicle stability.

## Claims

1. A vesicle-silica composite-containing composition comprising:
(A) a surfactant which can form vesicles in water and
(B) water, and containing a vesicle-silica composite wherein a part or a whole of outer surface of the vesicles composed of the (A) surfactant is coated with silica.

2. The vesicle-silica composite-containing composition according to claim 1,
wherein the (A) surfactant is a polyoxyalkylene-modified silicone.

3. The vesicle-silica composite-containing composition according to claim 1 or 2,
wherein the amount of the (A) surfactant is 0.3 to 5.0 % by mass with respect to the total amount of the composition.

4. The vesicle-silica composite-containing composition according any of claim 1 to 3, containing a vesicle-silica composite particle having two or more vesicle particles in an aggregated state bonded by silica.

5. A production method of a vesicle-silica composite-containing composition comprising the steps of:
mixing ingredients containing (A) a surfactant which can form vesicles in water and (B) water to form vesicles composed of the (A) surfactant, and
adding (C) a water-soluble silane derivative represented by following formula (1) to the vesicle-containing aqueous composition obtained in the previous step to form silica on a part or a whole of outer surface of the vesicles composed of the (A) surfactant,
Si-(OR¹)₄ (1)
in the formula (1), at least one of R¹s is a polyhydric alcohol residue and the rest can be alkyl groups.

6. The production method of the vesicle-silica composite-containing composition according to claim 5, wherein the (A) surfactant is a polyoxyalkylene-modified silicone.

7. The production method of the vesicle-silica composite-containing composition according to claim5 or 6, wherein the amount of the (A) surfactant is 0.3 to 5.0 % by mass with respect to the total amount of the composition.

8. The production method of the vesicle-silica composite-containing composition according to any of claim 5 to 7, wherein the amount of the (C) water-soluble silane derivative is 1.0 to 10.0 % by mass with respect to the total amount of the composition.

9. The production method of the vesicle-silica composite-containing composition according to any of claim 5 to 8, wherein a vesicle-silica composite particle having two or more vesicle particles in an aggregated state bonded by silica is formed.

10. A cosmetic comprising the vesicle-silica composite-containing composition according to any of claim 1 to 4.

11. A fragrance cosmetic comprising the vesicle-silica composite-containing composition according to any of claim 1 to 4.
